# EUROPEAN PATENT SPECIFICATION

(11) **EP 3 706 816 B1**
(45) Date of publication and mention of the grant of the patent: **04.06.2025**
(21) Application number: 18812444.0
(22) Date of filing: 05.11.2018
(51) Int. Cl.: A61L 29/08, A61L 29/14, A61L 26/00

(54) **RADIATION STERILIZED HYDROGELS, MEDICAL DEVICES INCLUDING RADIATION STERILIZED HYDROGELS AND METHODS OF MAKING THE SAME**
STRAHLUNGSSTERILISIERTE HYDROGELE, MEDIZINISCHE VORRICHTUNGEN MIT STRAHLUNGSSTERILISIERTEN HYDROGELEN UND VERFAHREN ZU IHRER HERSTELLUNG
HYDROGELS STÉRILISÉS PAR RAYONNEMENT, DISPOSITIFS MÉDICAUX COMPRENANT DES HYDROGELS STÉRILISÉS PAR RAYONNEMENT ET LEURS PROCÉDÉS DE FABRICATION

(30) Priority: 06.11.2017 US 201762582123 P
(43) Date of publication of application: 16.09.2020
(73) Proprietor: Hollister Incorporated, Libertyville, IL 60048 (US)
(72) Inventor: FARRELL, David, J., Libertyville IL 60048 (US); PANESAR, Satwinder, Libertyville IL 60048 (US)
(74) Representative: FRKelly
(86) International application number: PCT/US2018/059235
(87) International publication number: WO 2019/090239

(56) References cited:
- CN-B- 104 474 583
- US-A1- 2008 152 698
- US-A1- 2010 055 153
- BLASQUES BUENO VANIA ET AL: "Synthesis and swelling behavior of xanthan-based hydrogels", CARBOHYDRATE POLYMERS, vol. 92, 1 November 2012 (2012-11-01), pages 1091 - 1099, XP093265521
- DATABASE WPI Week 201540, Derwent World Patents Index; AN 2015-32139M, XP002788963

## Description

The present application claims the benefit of and priority to U.S.

Provisional Patent Application No. 62/582,123, filed November 6, 2017.

### DESCRIPTION

### TECHNICAL FIELD

The present disclosure generally relates to radiation sterilized hydrogels that have a high water content and remain physically stable after being irradiated. The present disclosure also relates to medical devices that incorporate such hydrogels and methods of making such hydrogels.

### BACKGROUND

Several medical devices use or include hydrogels for a variety of different reasons. Hydrogels are water-insoluble polymers that have the ability to swell in water or aqueous solution without dissolution and to retain a significant portion of water or aqueous solution within the hydrogel structure. Hydrogels may possess a degree of flexibility similar to natural tissue and may be highly lubricious, due to their significant water content. When used in a medical device, hydrogels may be used to provide a lubricious surface, tissue compatibility, drug release, tissue replacement, etc. Such medical devices may include urinary catheters, wound care dressings, endotracheal tubes, stents, vascular catheters, etc.

CN104474583A discloses a sheet-like hydrogel material and preparation method thereof comprising 4-18 wt% polyvinyl alcohol, 0-12 wt% polyvinylpyrrolidone, 0-2 wt% polyethylene glycol, 0-3 wt% sodium alginate, 0-0.5 wt% chitosan, 0-1.5 wt% xanthan, 0-1.5 wt% sodium polyacrylate and 69.9-93.5 wt% water. The raw materials are heated up while stirring to provide a transparent, thick solution, cooled between a two-layer mold, shaped and packaged, then radiation sterilized. The radiation can be electron beam radiation with a dose of 5-20 kGy.

It is typically desirable, and in certain instances required, for medical devices to be sterilized during manufacturing and packaging. For efficiency and costs, it is desirable to sterilize medical devices with sterilizing radiation, such as gamma or E-beam radiation. One issue with hydrogels is that they are generally considered fragile or unstable when irradiated. This is especially an issue for hydrogels having high water content. This radiation instability can prevent the use of radiation sterilization processes for medical devices that include a fragile hydrogel.

Therefore, there remains a need for hydrogels that can be sterilized by radiation and substantially retain their physical properties.

### SUMMARY

There are several aspects of the present subject matter which may be embodied separately or together in the devices, compositions, methods and systems described herein and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a radiation sterilized hydrogel includes a hydrocolloid in an amount of below 6 wt%, the hydrocolloid comprising gellan gum, high acyl gellan gum, low acyl gellan gum or combinations thereof; a carboxylic acid in an amount of above 0.05 wt%, wherein the carboxylic acid comprises citric acid; and water in an amount above about 94 wt%.

In another aspect, not claimed, a radiation sterilized urinary catheter includes a catheter shaft adapted for insertion into the urethra of a patient and a lubricious hydrogel disposed on the outer surface of the catheter shaft. The lubricous hydrogel includes a hydrocolloid, a carboxylic acid, and water.

In another aspect, not claimed, a ready to use medical device assembly that includes a gas impermeable package containing a medical device and a hydrogel. The medical device has a hydrophilic surface. The hydrogel includes water, wherein the water in the hydrogel releases a water vapor within the gas impermeable package to form a vapor atmosphere in the gas impermeable that activates at least a portion of the hydrophilic surface.

In another aspect, not claimed, a wound dressing includes a radiation sterilized hydrogel comprising a hydrocolloid, a carboxylic acid, and water.

In another aspect, not claimed, a method of forming a urinary catheter is disclosed. The method includes forming a mixture of hydrocolloid, carboxylic acid and water. The mixture is then heated to form or retain the mixture in a liquid state. The catheter shaft is then dipped into the mixture to dispose the mixture on a surface of the catheter shaft. The mixture disposed on the surface of the catheter is then cooled to form a hydrogel on the surface of the catheter shaft. The catheter, having the hydrogel disposed thereon, is sterilized with radiation.

In another aspect, not claimed, a method of forming a hydrogel includes forming a mixture of hydrocolloid, carboxylic acid and water and heating the mixture to form or retain the mixture in a liquid state. The mixture is then cooled to form a hydrogel and the hydrogel is exposed to sterilizing radiation.

### BRIEF DESCRIPTION OF FIGURES

Fig. 1 is a cross-sectional view of a catheter shaft having a hydrogel disposed on the surface of the catheter shaft;
Fig. 2 is a top plan view of a package including a hydrophilic catheter and a radiation stable hydrogel; and
Fig. 3 is a cross-sectional view of the package of Fig. 2 taken along line 3-3.

### DETAILED DESCRIPTION OF THE ILLUSTRATED EMBODIMENTS

The embodiments disclosed herein are for the purpose of providing a description of the present subject matter, and it is understood that the subject matter may be embodied in various other forms and combinations not shown in detail. Therefore, specific embodiments and features disclosed herein are not to be interpreted as limiting the subject matter as defined in the accompanying claims.

The present disclosure relates to hydrogels that may be used as or incorporated into medical devices or device assemblies. Such hydrogels may be radiation stable hydrogels that substantially retain their properties after being exposed to radiation, such as gamma or E-beam radiation, which may be used to sterilize the hydrogel and/or medical device including such hydrogels. Furthermore, the hydrogels disclosed herein may remain stable after receiving a dose of radiation of between 25 kGy and about 45 kGy. The hydrogels may also be stable after receiving higher or lower doses of radiation as well. Such hydrogels may be radiation stable in that they maintain their physical properties or have little degradation during and after be exposed to radiation. The radiation stable hydrogels disclosed herein may be a high water content hydrogel which has above about 90 wt% of water within the hydrogel.

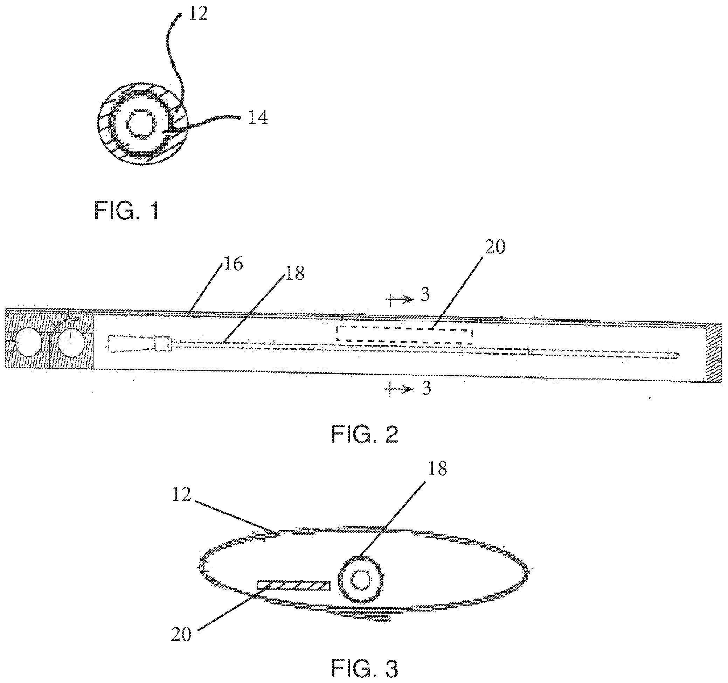

As mentioned above, the hydrogels disclosed herein may themselves be used as a medical device or the hydrogels may be incorporated into a medical device. When the hydrogels are used as a medical device, the hydrogels may be, for example, wound care dressings that are applied to a wound, such as burn wounds, necrotic wounds, pressure ulcers, donor sites, etc. The hydrogels also may be disposed on a medical device to provide, for example, a lubricous surface and/or tissue compatible surface. Referring to Fig. 1, in one embodiment, the hydrogel 12 may be disposed on the surface of a urinary catheter 14 to provide a lubricious surface that eases insertion of the catheter into and withdrawal of the catheter from the patient.

In further use, the hydrogels disclosed herein may be used to create a vapor atmosphere within a medical device package. For example, referring to Figs. 2 and 3, a package 16 may include a medical device (such as a catheter 18) that requires, or wherein it is beneficial to have, a vapor atmosphere or humid atmosphere in the package 16 during storage and distribution. In such instances, the hydrogel 20 may be placed within the package 16. The package 16, having the catheter and hydrogel therein, may then be exposed to sterilizing radiation. The sterilizing radiation may be gamma or E-beam and may be at a dose between about 25 kGy and about 45 kGy. After being irradiated, the water within the hydrogel provides a vapor that is released from the hydrogel to form a vapor atmosphere within the package. For example, the package 16 may be a gas impermeable package that includes a hydrophilic catheter 18 having a hydrophilic surface and a hydrogel 20 that releases vapor. The vapor hydrates or activates the hydrophilic surface, such that the catheter is ready to use right out of the package. In one embodiment, the catheter 18 may include a hydrophilic coating that becomes lubricious when hydrated or active by water vapor within the package.

In one embodiment, the hydrogel may be a hydrocolloid based hydrogel. The hydrocolloid(s) may include, but are not limited to, gellan gum, high acyl gellan gum, low acyl gellan gum and combinations thereof. The concentration of the hydrocolloid(s) of the hydrogel may be less than about 6 wt%. In one embodiment, the hydrocolloid(s) may be between about 0.1 wt% to 6 wt%, preferably 1 wt% to 2 wt% and more preferably 1.25 wt% and about 1.75 wt%. The amount of hydrocolloid may be adjusted depending on the desired use and conditions. For example, if the hydrogel is exposed to high doses of radiation, the amount of hydrocolloid may be increased, and if the hydrogel is exposed to a lower dose of radiation, the amount of hydrocolloid may be decreased.

The hydrogel may also include a gel-strengthening composition, such as carboxylic acid. The carboxylic acid may include, but is not limited to, citric acid. The gel-strengthen agent may be in an amount above 0.05 wt% of the hydrogel. In one embodiment, the gel-strengthening agent may be between about 0.05 wt% and about 0.5 wt% of the hydrogel.

The hydrogel may be a high water content hydrogel wherein the water content is above about 94 wt% of the hydrogel. In one embodiment, the water content may be above 97 wt% or above 98 wt%. In another embodiment, the water content may be between about 97 wt% and about 98.5 wt%. In one embodiment, the hydrogel may be between about 97 wt% and 98.5 wt% water and the remainder a mixture of hydrocolloid and strengthening agent (solids).

In one of embodiment of a radiation stable hydrogel, the hydrogel may include a hydrocolloid (such as gellan gum) in an amount of below 2 wt%, a carboxylic acid in an amount of above 0.05 wt%, and water in an amount above about 94 wt% and preferably about 97 wt%. In another embodiment, the hydrogel may include a hydrocolloid (such as gellan gum) in an amount between about 0.1 wt% to 6 wt%, preferably about 1 wt% to 2 wt% and more preferably about 1.25 wt% to about 1.75 wt% of hydrogel, between about 0.05 wt% and about 2 wt% of carboxylic acid (i.e. citric acid), and between about 94 wt% and about 98.5 wt% water.

The hydrogel may include other additives, such as stain-reducing additives, lubricious additives, etc. The hydrogel may include one or more of such additives. In one embodiment, the stain-reducing additive may be an additive that reduces staining of a fabric or other material that comes into contact with the hydrogel. The stain-reducing additive may mask or act as a clarifying agent. For example, when a hydrogel comes into contact with a fabric, the water of the hydrogel may be transferred to the fabric, leaving a stain on the fabric. The stain-reducing additive may include a polyol, such as a low molecular polyol. The concentration of the stain-reducing additive may be greater than about 0.25 wt% of the hydrogel. In one embodiment, the stain-reducing additive may be between about 0.25 wt% and about 0.5 wt% and about 5 wt%. In one embodiment, the polyol may be one or more of glycerol, polyethylene glycol, and xylitol.

In another embodiment, the additive may be a lubricious additive. For example, the lubricious additive may be a hydrophilic monomer or polymer. The hydrophilic polymer may be a crosslinked hydrophilic polymer. Such hydrophilic polymers and monomers may include polyvinylpyrrolidone (PVP), polyethylene oxide, polyurethanes, homo- and copolymers of acrylic and methacrylic acid, polyvinyl alcohol, polyvinyl ethers, maleic anhydride based copolymers, polyesters, vinyl amines, polyethylenimines, polyethylene oxides, poly(carboxylic acids), polyamides, polyanhydrides, polyphosphazenes, cellulosics, for example methyl cellulose, carboxymethyl cellulose, hydroxymethyl cellulose, and hydroxypropyl cellulose, other polysaccharides. In one embodiment, the hydrogel (in the dry state) may include hydrophilic polymer(s) in an amount greater than about 80 wt% of the non-aqueous components.

The hydrogels disclosed herein also may have a melting point above about 70°C. In one method of forming a hydrogel, hydrocolloid(s), gel-strengthening agent(s) and water are mixed together to form a mixture. Optionally, additives are added to the mixture. For example, water as a solvent (94 wt% - 98.75 wt%) is heated to a temperature above which the hydrocolloid (e.g. gellan gum or polysaccharide) melts or fully dissolves in the water. The hydrocolloid, which is typically in a powder form, is added at 0.1 wt% to 6%wt (preferably 1.25 wt% -1.75 wt%) into the heated water, which has been heated to at or above the hydrocolloids dissolution temperatures (which may be greater than 41°C and preferably is 60°C - 75°C). The hydrocolloid may be fully dispersed in solution and fully dissolved in solution to form a homogeneous solution. The dispersion and dissolution may be attained by mixing aids such as a low shear homogenizer or stirrer. Other ingredients, such as glycerol (0 - 0.5 wt%), citric acid (0 - 0.5 wt%) or a hydrophilic polymer may optionally be added to the heated solution and are dispersed and dissolved in solution by the mixing aids. The heated solution is then cooled to form the hydrogel. In one embodiment, for example, when the solution is cooled to temperature below 45°C, it forms a hydrogel.

Prior to forming the hydrogel, the heated solution may be cast or otherwise formed into a desired shape, and then cooled to thereby form a hydrogel having the desired shape. In one embodiment, the solution is cast into sheets and then cooled to form sheets of hydrogel. The sheets may then be cut or stamped into desired shapes, such as cubes or cylinders. The hydrogels may then be packaged and/or irradiation sterilized.

When the hydrogel is formed or disposed on the surface of a medical device, the heated solution is disposed on the medical device and the mixture is cooled to form the hydrogel on the medical device. For example, the medical device may be dipped into the heated solution or the solution may be poured or brushed or sprayed onto the medical device. In one embodiment, a urinary catheter may be dipped into a heated bath of the solution to dispose the solution on the catheter. The solution on the catheter is then cooled to form a hydrogel on the surface of the catheter. In one embodiment, the hydrogel provides a lubricious surface on the catheter.

After the hydrogel has been formed, whether the hydrogel itself is a medical device or part of the medical device, the hydrogel may then be sterilized with radiation, as described above. In one embodiment, after the hydrogel has been formed on a urinary catheter as described above to provide a lubricous surface, the catheter may be placed in a package and the package having the catheter therein may be sterilized with radiation. In another embodiment wherein the hydrogel is provided to provide a hydration atmosphere within a package, the medical device (such a hydrophilic catheter) and the hydrogel are placed in the package. The package is then sealed and the package, medical device and hydrogel are sterilized with radiation. After sterilization, the hydrogel release/provides a vapor within the package.

In addition to the other properties and characteristics described herein, the radiation stable hydrogel may also provide an anti-fouling or anti-adhesion property that prevents or reduces adhesion of microbials. For example, the hydrogel may provide a carbohydrate functional surface that provides an anti-fouling property (i.e., prevents or reduces microbial adhesion). In one embodiment, a hydrogel having a polysaccharide or polysaccharide derivative may provide such anti-fouling properties. The polysaccharides and/or polysaccharide derivatives may be bio-exopolysaccharides or synthetic polysaccharides. Also, the polysaccharides and/or polysaccharide derivatives may be any of those described herein or any other suitable ones. Furthermore, the hydrogel may be one that is based on such polysaccharides and/or polysaccharide derivatives or the polysaccharides and/or polysaccharide derivatives may be an additive that is added to the hydrogel. In one embodiment, the hydrogel may include only one polysaccharide, such as gellan gum, or may be a mixture of polysaccharides and derivatives, such as gellan gum and other polysaccharides and derivatives. Also, the hydrogels may include any of the above-discussed additives (strengthening agents, anti-staining agents and lubricous polymers).

In one embodiment, a urinary catheter includes a lubricious hydrogel wherein the hydrogel also provides a carbohydrate functionalized surface having anti-fouling properties. The anti-fouling properties may be beneficial in the field of urinary catheters wherein there is a risk of the catheter carrying/transmitting bacteria through the urethra and into the bladder, thereby causing infection. The lubricious hydrogel having anti-fouling properties is beneficial because the hydrogel prevents or reduces the adhesion of microbials to the catheter, and thus, the catheter is less likely to carry or transmit bacteria through the urethra and into the bladder, thereby reducing the risk of bacterial infection.

## Claims

1. A radiation sterilized hydrogel comprising:
a hydrocolloid in an amount of below 6 wt%, the hydrocolloid comprising gellan gum, high acyl gellan gum, low acyl gellan gum or combinations thereof;
a carboxylic acid in an amount of above 0.05 wt%, wherein the carboxylic acid comprises citric acid; and
water in an amount above about 94 wt%.

2. The sterilized hydrogel of claim 1 wherein the amount of hydrocolloid is about 0.1 wt% to 6 wt%, preferably 1 wt% to 2 wt% and more preferably between about 1.25 wt% and about 1.75 wt%.

3. The sterilized hydrogel of any one of claims 1-2, wherein the amount of the carboxylic acid is between about 0.05 wt% and about 0.5 wt%.

4. The sterilized hydrogel of any one of claims 1-3, further including an additive comprising a stain-reducing additive.

5. The sterilized hydrogel of any one of claims 1-4, further including an additive comprising a polyol.

6. The sterilized hydrogel of claim 5, wherein the polyol comprises one or more of glycerol, polyethylene glycol and xylitol.

7. The sterilized hydrogel of any one of claims 1-6, furthering including an additive comprising a lubricious additive.

8. The sterilized hydrogel of claim 7, wherein the lubricious additive comprises a hydrophilic polymer.

9. The sterilized hydrogel of claim 8, wherein the hydrophilic polymer is crosslinked.

10. The sterilized hydrogel of any one of claims 8 and 9, wherein the hydrophilic polymer comprises polyvinylpyrrolidone.

11. The sterilized hydrogel of any one of claims 1-10, wherein the hydrogel is sterilized by gamma radiation.

12. The sterilized hydrogel of any one of claims 1-10, wherein the hydrogel is sterilized by E-beam radiation.

13. The sterilized hydrogel of any one of claims 1-12, wherein the hydrogel is sterilized with a dose of radiation between about 25 kGy and about 45 kGy.

## Patentansprüche

1. Ein strahlungssterilisiertes Hydrogel, umfassend:
Hydrokolloid in einer Menge von unter 6 Gew.-%, wobei das Hydrokolloid Gellan, Hochacyl-Gellan, Niedrigacyl-Gellan oder Kombinationen davon umfasst;
eine Carbonsäure in einer Menge von über 0,05 Gew.-%, wobei die Carbonsäure Zitronensäure umfasst; und
Wasser in einer Menge von über etwa 94 Gew.-%.

2. Sterilisiertes Hydrogel nach Anspruch 1, wobei die Menge an Hydrokolloid etwa 0,1 Gew.-% bis 6 Gew.-%, vorzugsweise 1 Gew.-% bis 2 Gew.-% und noch bevorzugter zwischen etwa 1,25 Gew.-% und etwa 1,75 Gew.-% beträgt.

3. Sterilisiertes Hydrogel nach einem der Ansprüche 1-2, wobei die Menge der Carbonsäure zwischen etwa 0,05 Gew.-% und etwa 0,5 Gew.-% liegt.

4. Sterilisiertes Hydrogel nach einem der Ansprüche 1-3, ferner beinhaltend ein Additiv, umfassend ein schmutzreduzierendes Additiv.

5. Sterilisiertes Hydrogel nach einem der Ansprüche 1-4, ferner beinhaltend ein Additiv, umfassend ein Polyol.

6. Sterilisiertes Hydrogel nach Anspruch 5, wobei das Polyol eines oder mehrere von Glycerin, Polyethylenglykol und Xylit umfasst.

7. Sterilisiertes Hydrogel nach einem der Ansprüche 1-6, ferner beinhaltend ein Additiv, umfassend ein Gleitadditiv.

8. Sterilisiertes Hydrogel nach Anspruch 7, wobei das Gleitadditiv ein hydrophiles Polymer umfasst.

9. Sterilisiertes Hydrogel nach Anspruch 8, wobei das hydrophile Polymer vernetzt ist.

10. Sterilisiertes Hydrogel nach einem der Ansprüche 8 und 9, wobei das hydrophile Polymer Polyvinylpyrrolidon umfasst.

11. Sterilisiertes Hydrogel nach einem der Ansprüche 1-10, wobei das Hydrogel durch Gammastrahlung sterilisiert wird.

12. Sterilisiertes Hydrogel nach einem der Ansprüche 1-10, wobei das Hydrogel durch E-Beam-Strahlung sterilisiert wird.

13. Sterilisiertes Hydrogel nach einem der Ansprüche 1-12, wobei das Hydrogel mit einer Strahlungsdosis zwischen etwa 25 kGy und etwa 45 kGy sterilisiert wird.

## Revendications

1. Hydrogel stérilisé par rayonnement comprenant :
un hydrocolloïde en une quantité inférieure à 6 % en poids, l'hydrocolloïde comprenant de la gomme gellane, de la gomme gellane à haute teneur en acyle, de la gomme gellane à faible teneur en acyle ou des combinaisons de celles-ci ;
un acide carboxylique en une quantité supérieure à 0,05 % en poids, dans lequel l'acide carboxylique comprend de l'acide citrique ; et
de l'eau en une quantité supérieure à environ 94 % en poids.

2. Hydrogel stérilisé selon la revendication 1, dans lequel la quantité d'hydrocolloïde est comprise entre environ 0,1 % en poids et 6 % en poids, de préférence entre 1 % en poids et 2 % en poids et plus préférablement entre environ 1,25 % en poids et environ 1,75 % en poids.

3. Hydrogel stérilisé selon l'une quelconque des revendications 1 et 2, dans lequel la quantité d'acide carboxylique est comprise entre environ 0,05 % en poids et environ 0,5 % en poids.

4. Hydrogel stérilisé selon l'une quelconque des revendications 1 à 3, comportant également un additif comprenant un additif réduisant les taches.

5. Hydrogel stérilisé selon l'une quelconque des revendications 1 à 4, comportant également un additif comprenant un polyol.

6. Hydrogel stérilisé selon la revendication 5, dans lequel le polyol comprend un ou plusieurs éléments parmi le glycérol, le polyéthylène glycol et le xylitol.

7. Hydrogel stérilisé selon l'une quelconque des revendications 1 à 6, comportant également un additif comprenant un additif lubrifiant.

8. Hydrogel stérilisé selon la revendication 7, dans lequel l'additif lubrifiant comprend un polymère hydrophile.

9. Hydrogel stérilisé selon la revendication 8, dans lequel le polymère hydrophile est réticulé.

10. Hydrogel stérilisé selon l'une quelconque des revendications 8 et 9, dans lequel le polymère hydrophile comprend de la polyvinylpyrrolidone.

11. Hydrogel stérilisé selon l'une quelconque des revendications 1 à 10, dans lequel l'hydrogel est stérilisé par rayonnement gamma.

12. Hydrogel stérilisé selon l'une quelconque des revendications 1 à 10, dans lequel l'hydrogel est stérilisé par rayonnement de faisceau électronique.

13. Hydrogel stérilisé selon l'une quelconque des revendications 1 à 12, dans lequel l'hydrogel est stérilisé avec une dose de rayonnement comprise entre environ 25 kGy et environ 45 kGy.
